# EUROPEAN PATENT APPLICATION

(11) **EP 3 431 091 A1**
(43) Date of publication of application: **23.01.2019**
(21) Application number: 17765790.5
(22) Date of filing: 10.03.2017
(51) Int. Cl.: A61K 31/7076, A61P 9/10, A61P 29/00

(54) **USE OF TRIACETYL-3-HYDROXYPHENYLADENOSINE IN PREPARING PHARMACEUTICALS FOR TREATMENT OF ATHEROSCLEROSIS**

(30) Priority: 14.03.2016 CN 201610141228
(71) Applicant: Jiangsu Tasly Diyi Pharmaceutical Co., Ltd., Huai'an, Jiangsu 223003 (CN); Institute of Materia Medica, Chinese Academy of Medical Sciences, Beijing 100050 (CN)
(72) Inventor: ZHU, Haibo, Huai'an Jiangsu 223003 (CN); WANG, Jing, Beijing 100050 (CN)
(74) Representative: Gulde & Partner
(86) International application number: PCT/CN2017/076304
(87) International publication number: WO 2017/157248

(57) **Abstract**

Provided are uses of an adenosine derivative, shown in formula (I), in preparing pharmaceuticals for the prevention or/and treatment of hyperlipidemia-associated monocytosis, and for the prevention or/and treatment of atherosclerosis. The prevention or/and treatment of atherosclerosis refers to the prevention or/and treatment of atherosclerosis-associated inflammatory monocytosis.

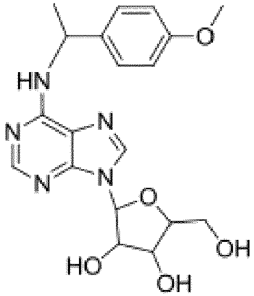

## Description

### Technical Field

The invention relates to uses of triacetyl-3-hydroxyphenyladenosine and a pharmaceutical composition containing the same in preparing pharmaceuticals for the prevention or/and treatment of hyperlipidemia-associated monocytosis and for the prevention or/and treatment of atherosclerosis, which belongs to the technical field of medicine.

### Background Art

Whether in Western developed countries or in China, cardiovascular diseases have already become the number one killer of human health and life due to changes in the eating habits and living standards of people.

Atherosclerosis is one of the primary pathological bases of cardiovascular and cerebrovascular diseases. During the formation of atherosclerosis, it is always accompanied by aggravated immune response and inflammatory reaction, and lymphocytes and lipids are accumulated in the aortic intima. Mononuclear cells are induced by inflammatory factors such as lipid-rich macrophages-foam cells and secreted inflammatory mediators MCP-1 and IL-6 to be migrated into vascular endothelium to stimulate smooth muscle cell migration and proliferation, and participate in plaque formation, development, rupture, and thrombosis.

Mononuclear cells, as intermediate cells, are continuously produced in the bone marrow, circulated in the blood and migrated into tissues, and then are further differentiated into macrophages, dendritic cells, etc. ApoE^{-/-} mice fed with high-fat diet, as compared with being fed with chow diet, the number of the mononuclear cells in the circulating blood has increased by 4 times, and the number of the inflammatory phenotype Ly6C^{hi} mononuclear cells has increased by 14 times, which significantly increases the number of macrophages in atherosclerotic plaques. It has been found in the studies on the mice suffering from atherosclerosis that bone marrow-derived inflammatory mononuclear cells are precursors of inflammatory macrophages and constitute major components of arterial plaque, and a large number of studies have shown that the mononuclear cells/macrophages are activated in atherosclerosis. Ox-LDL is continuously phagocytized by the macrophages and developed into foam cells, and formation of the foam cells is an important indicator of early atherosclerosis. The inflammatory signaling pathway is thus activated and then exacerbates the inflammatory response. Therefore, the inflammatory mononuclear cells play an important role in the occurrence and development of atherosclerosis.

Triacetyl-3-hydroxyphenyladenosine (Patent No. ZL200980101131.6, Publication No. CN101874036B, and Publication Date January 25th, 2012) is a new structure type compound with significant blood lipid regulating activity selected by the Institute of Materia Medica (IMM) at Chinese Academy of Medical Sciences (CAMS) from the cordycepin derivatives, and the compound is low in toxicity and good in pharmacokinetics and is currently in preclinical study stage. There has been no report on the use of this compound in the treatment of atherosclerosis-associated inflammatory mononucleosis.

### Summary of the Invention

The technical problem to be solved by the present invention is to provide uses of triacetyl-3-hydroxyphenyladenosine as shown in formula (I) in preparing pharmaceuticals for the prevention or/and treatment of hyperlipidemia-associated monocytosis and for the prevention or/and treatment of atherosclerosis.

To solve the technical problems of the present invention, the present invention provides the following technical solutions:
A first aspect of the technical solution of the present invention provides uses of triacetyl-3-hydroxyphenyladenosine as shown in formula (I) in preparing pharmaceuticals for the prevention or/and treatment of hyperlipidemia-associated monocytosis,

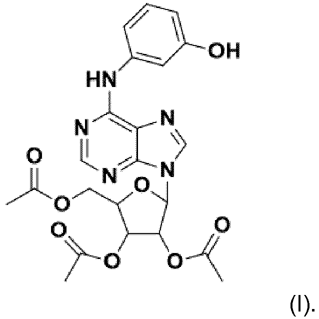

A second aspect of the technical solution of the present invention provides uses of triacetyl-3-hydroxyphenyladenosine as shown in formula (I) in preparing pharmaceuticals for the prevention or/and treatment of atherosclerosis,

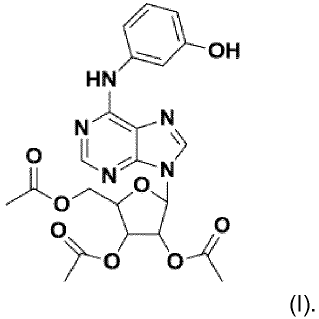

Further, the prevention or/and treatment of atherosclerosis refers to prevention or/and treatment of atherosclerosis- associated inflammatory monocytosis.

Wherein, the treatment of atherosclerosis with triacetyl-3-hydroxyphenyladenosine refers to a process in which the inflammatory mononuclear cells can be migrated from the bone marrow into the circulating blood. The significant increase in atherosclerosis-associated circulating blood inflammatory mononuclear cells can be improved, the number of circulating blood Ly6C^{hi} inflammatory mononuclear cells can be reduced, the level of serum MCP-1 inflammatory chemokines can be reduced, the inflammatory response can be reduced, and thus the formation of arterial plaques can be reduced.

The significant effect of triacetyl-3-hydroxyphenyladenosine in the treatment of atherosclerosis-associated monocytosis was demonstrated by the present invention with pharmacodynamics researching methods, so that there is scientific basis for the clinical application of triacetyl-3-hydroxyphenyladenosine in the treatment of atherosclerosis-associated monocytosis.

A third aspect of the technical solution of the present invention provides uses of a pharmaceutical composition in preparing pharmaceuticals for the prevention or/and treatment of hyperlipidemia-associated monocytosis, and in preparing pharmaceuticals for the prevention or/and treatment of atherosclerosis. Wherein the prevention or/and treatment of atherosclerosis refers to prevention or/and treatment of atherosclerosis-associated inflammatory monocytosis. The pharmaceutical composition comprises triacetyl-3-hydroxyphenyladenosine according to the first aspect and a pharmaceutically-acceptable carrier or additive.

The pharmaceutical composition can be prepared according to methods known in the art. The compound of the present invention may be combined with one or more pharmaceutically-acceptable solid or liquid excipients or/and adjuvants to be prepared into any dosage forms suitable for human or animal use. The content of the compound of the present invention in its pharmaceutical composition is usually from 0.1 to 95% by weight.

The compound of the present invention or the pharmaceutical composition containing the same can be administered in unitary dosage form intestinally or parenterally, such as through oral administration, intravenous injection, intramuscular injection, subcutaneous injection, the nasal cavity, the oral mucosa, the eyes, the lung and the respiratory tract, the skin, the vagina, and the rectum, etc.

Dosage forms can be liquid, solid or semi-solid dosage forms. The liquid dosage forms may be solutions (including true solutions and colloidal solutions), emulsions (including o/w type, w/o type, and multiple emulsions), suspensions, injections (including water injections, powder injections, and infusions), eye drops, nose drops, lotions and tinctures. The solid dosage forms may be tablets (including conventional tablets, enteric-coated tablets, buccal tablets, dispersible tablets, chewable tablets, effervescent tablets, orally disintegrating tablets), capsules (including hard capsules, soft capsules, and enteric-coated capsules), granules, powders, microcapsules, drop pills, suppositories, films, patches, aerosols, sprays, etc.; the semi-solid dosage forms can be ointments, gels, pastes, etc. The preferred dosage forms of the pharmaceutical composition are selected from the group consisting of tablets, capsules, pills, and injections.

The compound of the present invention can be prepared into conventional preparations, or sustained release preparations, controlled-release preparations, targeting preparations, and various microparticles delivery systems.

In order to prepare the compound of the present invention into tablets, various excipients known in the art can be widely used, including diluents, binders, wetting agents, disintegrants, lubricants, and gildants. The diluent may be starch, dextrin, sucrose, glucose, lactose, mannitol, sorbitol, xylitol, microcrystalline cellulose, calcium sulfate, calcium hydrogen phosphate, calcium carbonate, etc.; The wetting agent may be water, ethanol, isopropanol, etc.; the binder may be starch syrup, dextrin, syrup, honey, glucose solution, microcrystalline cellulose, acacia mucilage, gelatin slurry, sodium carboxymethylcellulose, methylcellulose, hydroxypropyl methylcellulose ethyecellulose, acrylic resin, carbomer, polyvinylpyrrolidone, polyethylene glycol, etc. The disintegrants may be dry starch, microcrystalline cellulose, low substituted hydroxypropyl cellulose, cross-linked polyvinylpyrrolidone, croscarmellose sodium, sodium carboxymethyl starch, sodium bicarbonate and citric acid, polyoxyethylene sorbitol fatty acid ester, and sodium dodecyl sulfate, etc. Lubricants and gildants may be talc, silica, stearate, tartaric acid, liquid paraffin, and polyethylene glycol, etc.

Tablets may also be further prepared into coated tablets, such as sugar-coated tablets, film-coated tablets, enteric-coated tablets, or double-layer tablets and multi-layered tablets.

To prepare the administration unit into a capsule, the active ingredient of the present invention may be mixed with a diluent, a glidant, and the mixture may be directly placed in a hard or soft capsule. The active ingredient of the present invention may also be granulated or pelletized with a diluent, a binder, or a disintegrant, and then placed in a hard or soft capsule. A wide variety of diluents, binders, wetting agents, disintegrants, and glidants used for the preparation of tablets of the compounds of the present invention may also be used in preparing capsules of the compounds of the present invention.

To prepare the compound of the present invention into an injection, water, ethanol, isopropyl alcohol, propylene glycol, or a mixture thereof may be added as a solvent to add an appropriate amount of a solubilizer, a co-solvent, a PH modifier, and an osmotic pressure regulator commonly used in the art. Solubilizers or glidants may be poloxamers, lecithins, hydroxypropyl-beta-cyclodextrins, etc.; PH modifier can be phosphate, acetate, hydrochloric acid, and sodium hydroxide, etc.; and the osmotic modifier may be sodium chloride, mannitol, glucose, phosphate, and acetate, etc. For preparing freeze-dried powder injections, mannitol, glucose, etc. may also be added as a proppant.

In addition, colorants, preservatives, perfumes, flavoring agents, or other additives may also be added to the pharmaceutical preparation as needed.

In order to achieve the purpose of administration and enhance the therapeutic effect, the pharmaceutical or pharmaceutical composition of the present invention may be administered by any known method of administration.

The dosage of administration of the pharmaceutical composition of the compound of the present invention may vary greatly depending on the nature and severity of the disease to be prevented or treated, the individual condition of the patient or animal, and the ways of administration and the dosage forms, etc. In general, suitable dosages per day for the compounds of the present invention will range from 0.001 to 150 mg/kg of body weight, preferably from 0.1 to 100 mg/kg of body weight, more preferably from 1 to 60 mg/kg of body weight, and most preferably from 2 to 30 mg/kg of body weight. The above dosages can be administered in one dosage unit or in several dosage units, depending on the clinical experience of the doctor and the dosing regimen that includes the use of other treatment means.

The compound or composition of the present invention may be administered alone or in combination with other therapeutic pharmaceuticals or symptomatic pharmaceuticals. When the compound of the present invention has a synergistic effect with other therapeutic pharmaceuticals, its dosage should be adjusted as actually required.

### Beneficial technical effects

The present invention provides a new therapeutic pharmaceutical triacetyl-3-hydroxyphenyladenosine for treating atherosclerosis which is a complicated pathological chronic disease with poor therapeutic effect. The therapeutic pharmaceutical has a significant curative effect in the treatment of atherosclerosis-associated inflammatory mononucleosis with small toxic side effects and safety.

### Brief Description of the Drawings

The invention will be further described with reference to embodiments of the present invention and the accompanying drawings in order to make the content of the present invention more clearly understood, in which,
FIG. 1 illustrates a comparison of the number of circulating blood mononuclear cells of the golden hamsters according to the experimental example of the present invention;

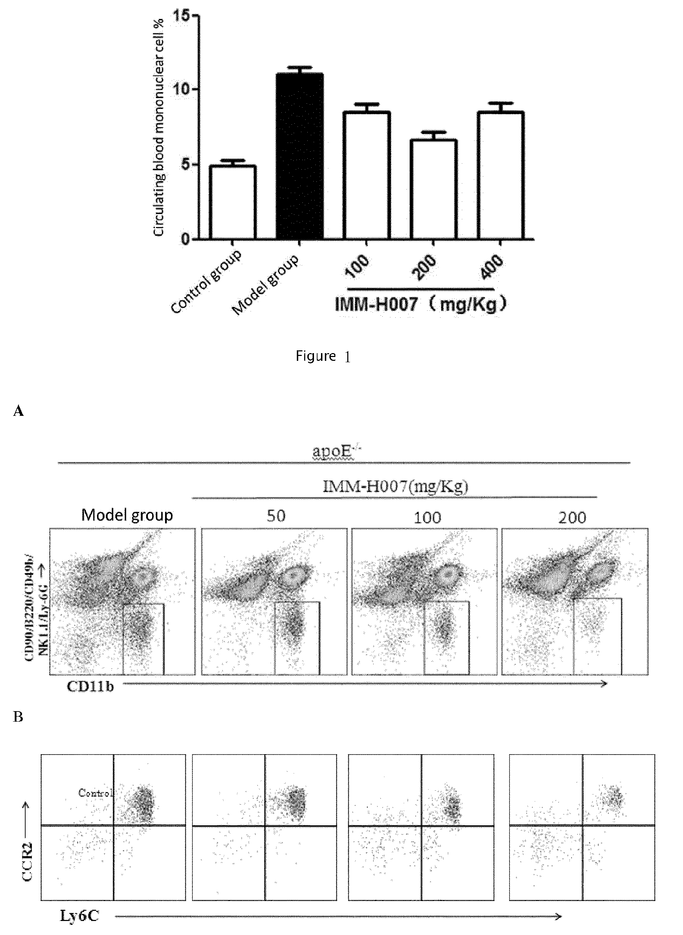

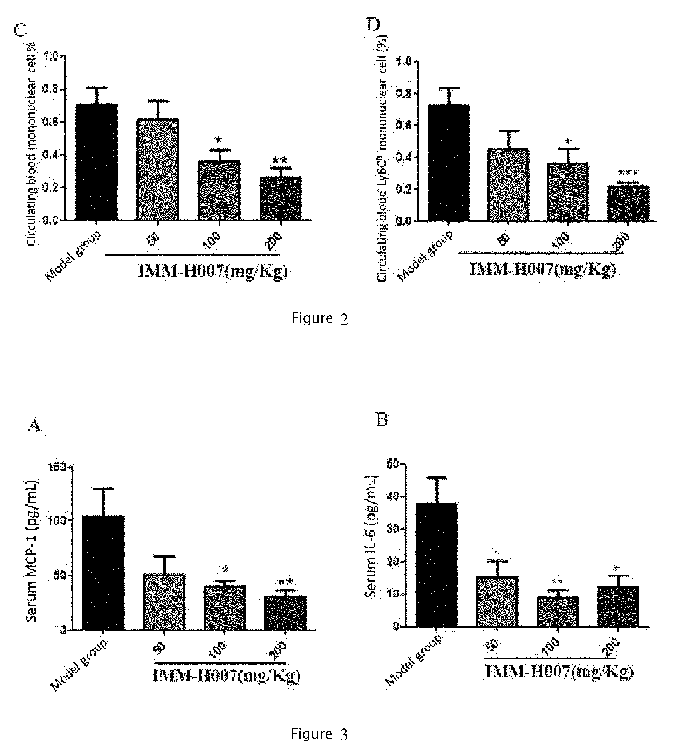

FIG. 2 illustrates a comparison of flow analysis results of apoE^{-/-} mice circulating blood mononuclear cells, inflammatory mononuclear cells, and resident phenotype mononuclear cells according to the experimental example of the present invention; peripheral blood Ly6C^{hi} mononuclear cells were reduced by IMM-H007. (A) High-fat diet-fed apoE-/-mice peripheral blood mononuclear cells were separated, and were marked with anti-CD11b, -CD90, -B220, -CD49b, -NK1.1, and -Ly-6G antibodies, and CD11b^{hi}CD90^{lo}B220^{lo}CD49b^{lo}NK1.1^{lo}Ly-6G^{lo} mononuclear cell ratio was subjected to phylum categorizing analysis; and (B) Ly-6C mononuclear cells and Ly-6C^{lo} mononuclear cells were further analyzed by Ly6C antibody labeling, and the cell ratio was represented by mean±SEM; (C) whole blood CD11b mononuclear cells (D) whole peripheral blood Ly6C^{hi} mononuclear cells;
FIG. 3 illustrates a comparison of serum levels of MCP-1 and IL-6 in apoE-/-mice according to the experimental example of the present invention;
FIG. 4 illustrates a comparison of the number of inflammatory mononuclear cells in arterial plaques of apoE-/-mice according to the experimental examples of the present invention;
FIG. 5 illustrates a comparison of the aortic roots and aortic full length oil red O staining in each group of apoE-/-mice according to the experimental example of the present invention.

### Detailed Description of the Invention

The following embodiments are used to further illustrate the present invention, but this does not mean any limitation to the present invention.

Experimental example 1: uses of Triacetyl-3-Hydroxyphenyladenosine (IMM-H007) in the treatment of hyperlipidemia- associated monocytosis.

### I. Experimental materials

### 1. Reagents

Abbott CD3700 whole blood cell analysis reagents, including diluents, hemolytic agents, sheath solutions, washing liquids, and enzyme cleaning solutions; EDTA-2K anticoagulant

### 2. Apparatus

Whole blood cell analyzer (Abbott, USA).

### 3. Experimental animal

Twenty Syrian golden hamsters (LVG hamsters, introduced from Charles River Laboratories) aged 6-8 weeks, weighing 90-120 g, male, SPF grade and purchased from Beijing Vital River Laboratory Animal Technology Co., Ltd.

### II. Experimental method

### 1. Animal grouping and feeding

After 7 days of adaptive feeding, animals were randomly divided into a normal control group (n=13), a high-fat feedstuff group (n=13), an IMMH007 low-dose group (50 mg/kg, n=13), an IMMH007 medium-dose group (100 mg/kg, n=13), and an IMMH007 high-dose group (200 mg/kg, n=13), and they were administered twice a day by gavage. The animals were fed in the Second Department of Animal Experiment Center of the Institute of Materia Medica (IMM) at Chinese Academy of Medical Sciences (CAMS); the feeding condition was SPF grade, the temperature was 21±2 DEG C, relative humidity was 50±5%, lighting cycle was 12/12, and 5 per cage. The normal group was fed with normal basal feedstuff, and the high-fat feedstuff group was fed with high fat feedstuff (79.8% of basal feedstuff, 20% of lard, and 0.2% of cholesterol), and the animals were free to take food and drink. The feedstuff was produced by Beijing HFK Bioscience Co., Ltd.

### 2. Observation of indicators and measurement methods

### 2.1 Circulating blood mononuclear cell measurement

The animals were fasted for 12 hours, and about 200 ul of blood was taken from the canthus of each eye, the blood was dissolved in 30ul of EDTA-2K anticoagulant (20g/L, prepared with normal saline), mixed slightly to avoid coagulation, and the whole blood cells were analyzed by a whole blood cell analyzer.

### III. Experimental results

Effect of IMM-H007 on circulating blood mononuclear cells of Syrian golden hamsters fed with high-fat diet.

From FIG. 1 and Table 1, it can be seen that compared with the model group, IMM-H007 can significantly reduce the number of mononuclear cells in the circulating blood of the Syrian golden hamsters.

**Table 1 Effect of IMM-H007 on the circulating blood mononuclear cells of the Syrian golden hamsters**

| Group(n=13) | Normal group | Model group | IMM-H007 (mg/Kg) | | |
|---|---|---|---|---|---|
| | | | 50 | 100 | 200 |
| | 3.99 | 15.2 | 9.21 | 6.8 | 8.52 |
| | 4.8 | 10.4 | 6.5 | 7.54 | 9.23 |
| | 5.42 | 10.7 | 8.91 | 4.56 | 5.7 |
| | 5.95 | 10.8 | 6.63 | 6.23 | 4.42 |
| | 2.26 | 10.9 | 5.31 | 4.18 | 6.33 |
| | 3.44 | 11.7 | 7.58 | 3.87 | 9.94 |
| | 6.04 | 10.3 | 11.2 | 6.05 | 9.51 |
| | 6.69 | 10.3 | 10.1 | 6.02 | 10.3 |
| | 3.4 | 11.2 | 11 | 7.99 | 6.28 |
| | 6.02 | 9.42 | 10.4 | 6.79 | 9.61 |
| | 5.79 | 12.1 | 9.08 | 9.61 | 9.85 |
| | 5.32 | 10.9 | 6.95 | 6.42 | 10.8 |
| | 4.9 | 10.4 | 8.24 | 10.4 | 10.3 |

Experimental example 2: uses of Triacetyl-3-Hydroxyphenyladenosine (IMM-H007) in the treatment of atherosclerosis- associated mouse model circulating blood monocytosis.

### I. Experimental materials

### 1. Reagents

OCT cryosection embedding agent, Sakura of America; Pentobarbital sodium, Sigma-Aldrich; PEG6000, Sigma-Aldrich; Glycine, Sigma-Aldrich; Paraformaldehyde, oil red O, Sigma-Aldrich; HE stained solution, Baso, Taiwan.

### 2. Apparatus

Multi-purpose low-temperature high-speed centrifuge, Eppendorff, Germany; Freezing microtome, Leica, Germany; Water-jacket thermostatic waterbath; En Vision multiplate reader, PerkinElmer, Inc., USA; Small animal anaesthetic machine, Matrx products, USA.

### 3. Experimental animal

ApoE-/-mice (C57BL background), 5-7 weeks old, body weight 18-22 g, male, SPF grade; Purchased from Beijing institute of Laboratory Animal Science.

### II. Experimental method

### 1. Animal grouping and feeding

After 7 days of adaptive feeding, animals were randomly divided into a high-fat feedstuff group (n=7), an MMH007 low-dose group (50 mg/kg, n=7), an IMMH007 medium-dose group (100 mg/kg, n=7), and an IMMH007 high-dose group (200 mg/kg, n=9), and they were administered once a day by gavage. The animals were fed in the Second Department of Animal Experiment Center of the Institute of Materia Medica (IMM) at Chinese Academy of Medical Sciences (CAMS); the feeding condition was SPF grade, the temperature was 21±2 DEG C, relative humidity was 50±5%, lighting cycle was 12/12, and 5 per cage. All groups were given high-fat diet (70% of basal diet plus 20% of lard and 0.2% of cholesterol) and the animals were free to take food and drink. The feedstuff was produced by Beijing HFK Bioscience Co., Ltd. Body weight was recorded once every week during the experiment.

### 2. Observation of indicators and measurement methods

### 2.1 Circulating blood mononuclear cell measurement

The animals were fasted for 12 hours and the following cells were extracted:
Recirculating blood cells: 50 ul of blood was taken by cardiocentesis, placed in 450 ul of heparin anticoagulant, 1 ml of red blood cell lysis buffer was added, lysed for 10 minutes, centrifuged at 1800r for 3 min, the supernatant was aspirated, and 100 ul of PBS suspension cells were obtained for subsequent use. The mouse was taken blood from the angular vein to be dead and serum was separated and stored at -80 DEG C.

Antibodies were added to the above blood cells:
3ul of CD90-PE, 3ul of B220-PE, 3ul of CD49b-PE, 3ul of NK1.1-PE, 3ul of Ly-6G-PE, 6ul of CD11b-percp, and 3ul of Ly-6C-FITC, a single positive tube and a blank control tube were provided, they were incubated with smoothie for 30min, washed 3 times with PBS, and
re-selected cells with 400 ul of PBS, filtered, and flow cytometry was carried out. The mononuclear cells were identified as CD11b^{hi}CD90^{lo}B220^{lo}CD49b^{lo}NK1.1^{lo}Ly-6G^{lo} cells.

According to the expression of Ly6C, Ly6C was highly expressed as LY6C^{hi} mononuclear cells in inflammatory mononuclear cells, and Ly6C was lowly expressed as Ly6Clo mononuclear cells in resident phenotype mononuclear cells.

After the spleen and bone marrow were taken from the mouse, the heart was slowly perfused with normal saline and the heart of the mouse was isolated and stored in 4% of paraformaldehyde.

### 2.2 Analysis of Ly6C^{hi} mononuclear cells in aortic full length arterial plaque

After the animals were fasted for 12 hours, the animals were taken blood from the canthus of the eyes, and were cut away the chest and abdomen under a stereomicroscope, saline was perfused through the heart and blood vessels. While keeping the blood vessels moist, the full length of the aorta was peeled and the adipose tissue of its surface was removed. The adipose tissue was separated, washed with PBS, added with mixed enzyme (125 U/ml of XI type collagenase, 60 U/ml of hyaluronidase, 60 U/ml of deoxyribose synthase, and 450 U/ml of type I collagenase, dissolved in 2.5 ml of PBS), the blood vessels were cut into pieces with scissors, incubated to be digested at 37 DEG C for 1 h, filtered with a 0.22 µm of filter membrane, centrifuged at 1600 rpm/3 min, discarded the supernatant, added with 100 µl of PBS, and added with antibodies:
3ul of CD90-PE, 3ul of B220-PE, 3ul of CD49b-PE, 3ul of NK1.1-PE, 3ul of Ly-6G-PE, 6ul of CD11b-percp, 3ul of Ly-6C-FITC, 10ul of F4/80-APC, 4ul of CD11C-APC, and 4ul of I-Ab-APC;
a single positive tube and a blank control tube were provided, they were incubated with smoothie for 30min, washed 3 times with PBS, and re-selected cells with 400 ul of PBS, filtered, and flow cytometry was carried out.

### 2.3 Analysis of serum inflammatory factors

Mouse serum inflammatory factors were analyzed with the BD flow mouse inflammatory factor measurement kit (Product No. 552364).

### 2.4 Aortic root and aortic full-length plaque area analysis

### 2.4.1 Preparation of cryosections

The temperature of the freezer of the microtome was set to -19 DEG C before cardiac tissue sectioning, and the sample head was set to -21 DEG C. The heart stored in 4% of paraformaldehyde was embedded in an OCT. Liquid nitrogen was rapidly frozen and the tissue was placed on a microtome sample table for temperature equilibration. After the tissue masses were repaired, they were serially sectioned with the thickness of the sections being 8 µm and they were attached to a clean polylysine-coated slide.

### 2.4.2 Oil red O staining

2.4.2.1 Oil red O staining of the cryosections: 5% of oil red O stock solution was prepared with isopropyl alcohol, then mixed evenly with water in a ratio of 3:2, filtered with a double layer filter paper, and added it to a staining tank. The selected sections were placed in a hanging basket, stained in a dyeing vat for 2 hours, subjected to color separation with 60% of isopropyl alcohol for a few seconds, washed slightly with tap water, counterstained with hematoxylin for 3-5 minutes, flushed with tap water for 2 minutes and placed back to the basket, sealed sections with glycerogelatin and observed and taken pictures under the stereomicroscope.
2.4.2.2 Aortic full length oil red O staining: the aortic full length was cut away, stained with oil red O for 4 h, rinsed with isopropyl alcohol, fixed with a stereomicroscope and taken pictures with a camera.

### 3. Data analysis

Data were represented as mean±standard error. All data were statistically analyzed through ONEWAY-ANOVA using a Graphpad Prism 5.0 software. The images were compared and analyzed.

### III. Experimental results

### 3.1 Effect of IMM-H007 on the circulating blood mononuclear cells of the mice suffering from atherosclerosis

As can be seen from FIG. 2, compared with the model group, IMM-H007 can significantly reduce the number of the circulating mononuclear cells in the apoE-/-mice (FIG. 2C, Table 2) and the number of inflammatory mononuclear cells (FIG. 2D, Table 3).

**Table 2 Effect of IMM-H007 on the circulating blood mononuclear cells of the apoE-/-mice**

| Group(n=7-9)) | Model group | IMM-H007 (mg/Kg) | | |
|---|---|---|---|---|
| | | 50 | 100 | 200 |
| | 0.38 | 0.63 | 0.29 | 0.23 |
| | 1.01 | 0.49 | 0.27 | 0.21 |
| | 0.41 | 0.26 | 0.25 | 0.10 |
| | 1.04 | 0.54 | 0.79 | 0.18 |
| | 0.76 | 0.49 | 0.48 | 0.69 |
| | 0.84 | 1.23 | 0.30 | 0.28 |
| | 0.49 | 0.66 | 0.14 | 0.18 |
| | | | | 0.24 |
| | | | | 0.27 |

**Table 3 Effect of IMM-007 on the circulating blood inflammatory mononuclear cells of the apoE-/-mice**

| Group(n=7-9) | Model group | IMM-H007 (mg/Kg) | | |
|---|---|---|---|---|
| | | 50 | 100 | 200 |
| | 0.78 | 0.89 | 0.30 | 0.15 |
| | 1.19 | 0.16 | 0.89 | 0.28 |
| | 1.06 | 0.49 | 0.26 | 0.21 |
| | 0.79 | 0.33 | 0.31 | 0.19 |
| | 0.73 | 0.86 | 0.19 | 0.35 |
| | 0.45 | 0.18 | 0.32 | 0.17 |
| | 0.38 | 0.23 | 0.29 | 0.20 |
| | | | | 0.30 |
| | | | | 0.13 |

### 3.2 Effect of IMM-H007 on serum inflammatory factor level of the mice suffering from atherosclerosis.

Effect of IMM-H007 on serum inflammatory factor level of the mice was further observed to find that IMM-H007 can significantly reduce the MCP-1 level (FIG. 3A) and IL-6 level (FIG. 3B) in serum.

### 3.3 Effect of IMM-H007 on the number of inflammatory mononuclear cells in the arterial plaques of the mice suffering from atherosclerosis

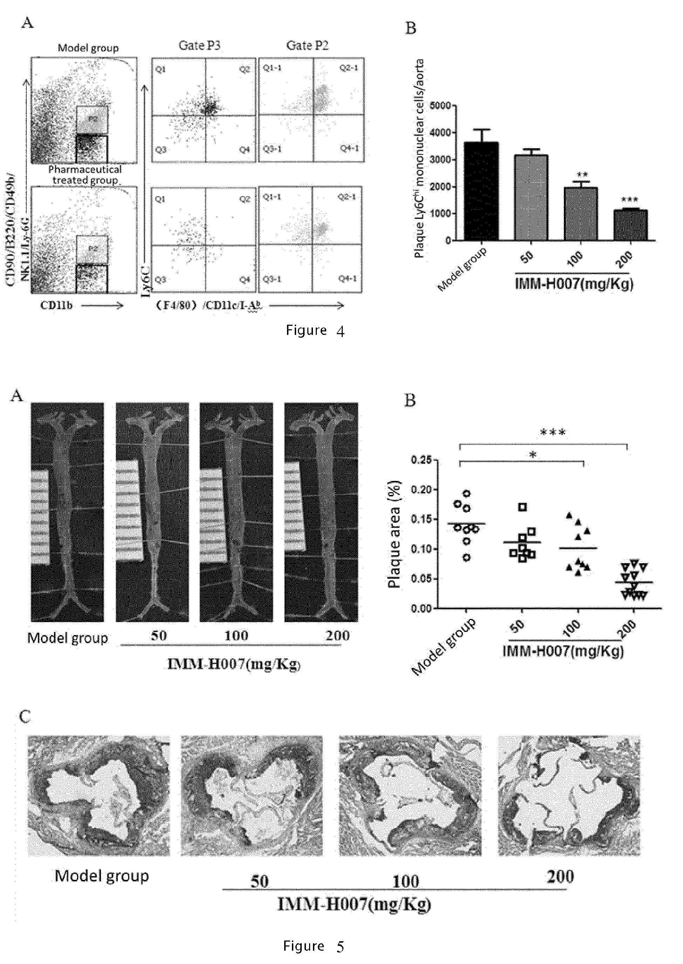

Effect of IMM-H007 on the number of inflammatory mononuclear cells in the arterial plaques of the mice was further observed to find that IMM-H007 can significantly reduce the number of inflammatory Ly6C^{hi} mononuclear cells in the arterial plaques (FIG. 4).

### 3.4 Effect of IMM-H007 on the arterial plaque formation of the mice suffering from atherosclerosis

It can be found through the oil red O staining of the aortic root and aortic full length of the apoE-/-mice that IMM-H007 can significantly reduce the arterial plaque area of the aortic full length and the aortic root of the apoE-/-mice. (FIG. 5).

## Claims

1. Uses of triacetyl-3-hydroxyphenyladenosine as shown in formula (I) in preparing pharmaceuticals for the prevention or/and treatment of hyperlipidemia-associated monocytosis,

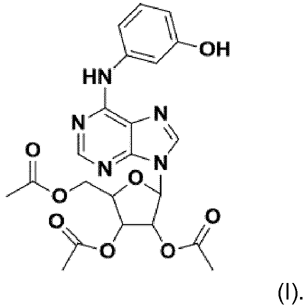

2. Uses of triacetyl-3-hydroxyphenyladenosine as shown in formula (I) in preparing pharmaceuticals for the prevention or/and treatment of atherosclerosis,

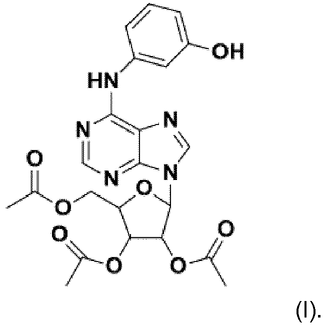

3. The uses according to claim 2, **characterized in that** the prevention or/and treatment of atherosclerosis refers to the prevention or/and treatment of atherosclerosis-associated inflammatory monocytosis.

4. The uses according to claim 2, **characterized in that** the prevention or/and treatment of atherosclerosis refers to capability in improving a significant increase in atherosclerosis-associated inflammatory mononucleosis, reducing the number of circulating blood Ly6C^{hi} inflammatory mononuclear cells, lowering the level of serum MCP-1 inflammatory chemokines, alleviating the inflammatory response, and reducing the formation of arterial plaques.

5. Uses of a pharmaceutical composition in preparing pharmaceuticals for the prevention or/and treatment of hyperlipidemia-associated monocytosis, **characterized in that** the pharmaceutical composition comprises triacetyl-3-hydroxyphenyladenosine as shown in formula (I) and pharmaceutically-acceptable carriers or excipients,

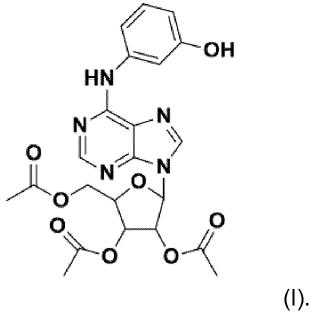

6. Uses of a pharmaceutical composition in preparing pharmaceuticals for the prevention or/and treatment of atherosclerosis, **characterized in that** the pharmaceutical composition comprises triacetyl-3-hydroxyphenyladenosine as shown in formula (I) and pharmaceutically-acceptable carriers or excipients,

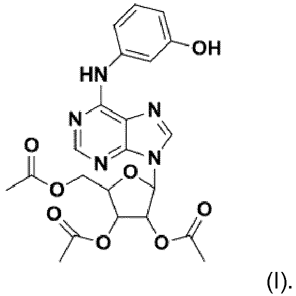

7. The uses according to claim 6, **characterized in that** the prevention or/and treatment of atherosclerosis refers to the prevention or/and treatment of atherosclerosis-associated inflammatory monocytosis.

8. The uses according to any one of claims 5-7, **characterized in that** the pharmaceutical composition is a tablet, a capsule, a pill or an injection.

9. The uses according to any one of claims 5-7, **characterized in that** the pharmaceutical composition is a sustained release preparation, a controlled release preparation or various microparticles delivery systems.
